# EUROPEAN PATENT APPLICATION

(11) **EP 4 279 905 A1**
(43) Date of publication of application: **22.11.2023**
(21) Application number: 22174532.6
(22) Date of filing: 20.05.2022
(51) Int. Cl.: G01N 21/78, G01N 21/84, G16H 10/40, G01N 21/77

(54) **METHOD OF INDIVIDUALIZING ANALYTE VALUE RANGES USED TO CATEGORIZE A CONCENTRATION OF AN ANALYTE**

(71) Applicant: F. Hoffmann-La Roche AG, 4070 Basel (CH); Roche Diabetes Care GmbH, 68305 Mannheim (DE)
(72) Inventor: HAILER, Fredrik, 68305 Mannheim (DE); BERG, Max, 68305 Mannheim (DE); LIMBURG, Bernd, 68305 Mannheim (DE)
(74) Representative: Kierig, Elisabeth

(57) **Abstract**

A method of individualizing analyte value ranges used to categorize a concentration of an analyte in a sample of a bodily fluid of a subject applied to a reagent test region and determined using a mobile device having a processing device, as well as a corresponding computer program, a non-transitory computer-readable storage medium with corresponding instructions, a corresponding mobile device, and a corresponding kit. The method comprises the steps of determining a numerical analyte result value from an image of a color formation of the reagent test region, attributing the numerical analyte result value to a corresponding analyte value range, and adapting one or more range limit values based on a plurality of numerical analyte result values previously determined for different samples of the subject's bodily fluid taken at different points in time.

## Description

### TECHNICAL FIELD

The present application refers to a method of individualizing analyte value ranges used to categorize a concentration of an analyte in a sample of a bodily fluid of a subject applied to a reagent test region and determined using a mobile device having a processing device. The method includes the steps of determining by the processing device from an image of a color formation of the reagent test region a numerical analyte result value within an overall measurement range and attributing the numerical analyte result value to a corresponding analyte value range of a series of consecutive analyte value ranges portioning the overall measurement range, each analyte value range with a range width extending between range limit values, each range limit value separating adjacent analyte value ranges;

The invention further relates to a computer program including computer-executable instructions for performing the method, a non-transitory computer-readable storage medium including instructions that when executed by a mobile device cause the mobile device to perform the method, a mobile device being configured for determining a concentration of an analyte in a bodily fluid, and a kit for determining a concentration of an analyte in a bodily fluid, the kit comprising at least one mobile device and at least one optical test strip having at least one test field.

### BACKGROUND

A number of different devices and methods for determining one or more analytes in body fluids, e.g. blood, urine, interstitial fluid and saliva, are known from prior art. Without narrowing the scope, the invention specifically will be described with respect to blood glucose measurements. It shall be noted, however, that the present invention may also be used for other types of analytical measurements using test elements.

Several test elements are known in the art which comprise at least one test chemical, also referred to as a test reagent, which undergo a coloration reaction in the presence of the at least one Analyte to be detected. Some basic principles on test elements and reagents that may also be used within the scope of the present invention are described e.g. in J. Hones et al.: Diabetes Technology and Therapeutics, Vol. 10, Supplement 1, 2008, pp. 10-26.

In analytical measurements based on color formation reactions, one technical challenge resides in the evaluation of the color change, which is due to the detection reaction. Besides using dedicated analytical devices, such as handheld blood glucose meters, the use of generally available electronics such as smart phones and portable computers has become more and more popular over the recent years. Such a method for detecting an analyte concentration using a mobile device is known from EP 3 591 385 A1.

WO 2019/157102 discloses a system and method for model based guidance of patients with diabetes. The model may for example include a physiology model or a behavior model.

US 2020/0367808 A1 discloses a postprandial monitoring device to assist in adjusting glucose levels by way of exercise. A measured glucose level is displayed by way of one or more categories the measured value fits into.

WO 2017/044654 A1 discloses calibration method for an Analyte concentration sensor as well as a method of compensating for drift in such a sensor. The calibration is based on prior known Analyte concentration values for specific repeatable events, e.g. a steady state.

US 10,548,537 B2 discloses a testing system for measuring a concentration of Analyte in a body fluid sample, wherein the system includes an input device for receiving information from a user relating to the measurements and a plurality of user-selectable statistical operations to provide the user with with enhanced information relating to the measurements. The information provided by the user may be categorized, e.g. with meal markers, and the categorization may be customized for different user groups, such as age groups.

When the user's target blood glucose range (e.g. 110-150mg/dl) is occasionally centered along an app category boundary, e.g. 131 mg/dl, then the user may get confused by getting perceived 'erratic' categories results (as the category may easily switch from measurement to measurement). Further user type specifics ('pre-diabetic, sportive, pre-existing conditions, etc., age, diabetic progression) may influence the target range and may even change on long term.

It is therefore desirable to provide methods and devices, which address the above-mentioned technical challenges of analytical measurements using mobile devices such as consumer-electronics mobile devices, specifically multipurpose mobile devices that are not dedicated to analytical measurements such as smart phones or tablet computers. Specifically, methods, computer programs, and devices shall be proposed, which are widely applicable to available mobile devices and which are suited to improve reliability and safety while allowing convenient handling for the user.

### BRIEF SUMMARY

This problem is addressed by methods, computer programs, and devices with the features of the independent claims. Advantageous embodiments which might be realized in an isolated fashion or in any arbitrary combination are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may all refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In one aspect, a method of individualizing analyte value ranges used to categorize a concentration of an analyte in a sample of a bodily fluid of a subject applied to a reagent test region and determined using a mobile device having a processing device is disclosed. The method includes the steps of determining by the processing device from an image of a color formation of the reagent test region a numerical analyte result value within an overall measurement range, attributing the numerical analyte result value to a corresponding analyte value range of a series of consecutive analyte value ranges portioning the overall measurement range, each analyte value range with a range width extending between range limit values, each range limit value separating adjacent analyte value ranges, adapting by the processing device or any other processing device one or more range limit values based on a plurality of numerical analyte result values previously determined for different samples of the subject's bodily fluid taken at different points in time.

"Analyte" refers to a substance or chemical constituent that is of interest in an analytical procedure and is also referred to as component or chemical species. As an example, one or more analytes may be determined which take part in metabolism, such as blood glucose. Additionally or alternatively, other types of analytes or parameters may be determined, e.g. a pH value. The analyte is comprised in at least one sample of a bodily fluid of a subject, i.e. a patient, and causes a test chemical, also referred to as a test reagent, to undergo a color formation reaction. To cause the reaction, the bodily fluid is applied to a reagent test region, the reagent test region, without limitation, may be part of a test strip or the like.

"Bodily fluid" refers to any liquid within the body, such as blood, interstitial fluid, urine, saliva or the like. "Color formation" refers to a color of a reagent test region resulting from a color formation reaction, i.e., a chemical, biological or physical reaction during which a color, specifically a reflectance, of at least one element involved in the reaction, changes with the progress of the reaction in the test region. Thereby, the concentration of the analyte in the bodily fluid may be determined by evaluating the color formation, i.e., the color change due to the reaction, in the reagent test region.

The method is based on determining the concentration of the analyte using a mobile device, wherein "mobile device" refers to a mobile electronics device, i.e., a portable device with at least one processing device, and, optionally, a display device and/or a camera. The mobile device may specifically refer to a mobile communication device such as a cell phone or smartphone. Additionally or alternatively, the mobile device may also refer to a tablet computer or another type of portable computer, optionally having a camera and/or any display device. "Processing device" refers to any device capable of processing data such as a data processor.

"Display device" refers to an output device for communication of information to a user in visual or tactile form, such as, without limitation, LCD displays, LED displays, or tactile electronics displays and which may serve to communicate a result such as a determined analyte value range and/or an associated message to the subject or any other person to whom it might be of importance. An associated or corresponding "message" refers to any displayable sign capable of informing the user of the status of the analyte concentration, i.e., of the determined analyte value range. The message may comprise one or more of a range name, corresponding values like the range limit values, a corresponding recommendation, a symbol, and the like.

"Camera" refers to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images.

Correspondingly, the term "image" relate to data recorded by using a camera, such as a plurality of electronic readings from the imaging device, such as the pixels of the camera chip. The image may be taken with a camera comprised by the mobile device. Alternatively, the image may be taken by any other camera and transferred to the mobile device via a wired or wireless communication path/interface.

The camera specifically may be a color camera. Thus, such as for each pixel, color information may be provided or generated, such as color values for three colors R, G, B. A larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The concentration of the analyte in a subject's bodily fluid is determined by the processing device of the mobile device based on an image of a reagent test region taken by the camera of the mobile device or another camera, wherein a sample of the subject's bodily fluid is applied to the reagent test region before taking the image causing the color formation. The color formation, i.e. the color of the reagent test region, in the image is determined by the processing device and a numerical analyte result value corresponding to the determined color formation is then identified by the processing device, wherein "numerical analyte result value" refers, to a numerical indication of an analyte concentration in the subject's bodily fluid sample.

The processing device then attributes a corresponding analyte value range to the numerical analyte result value determined in step (b) to communicate the measurement result in form of the analyte value range to the subject or any other person needing to know.

"Analyte value range" refers to a portion of a measurement range, i.e., the range of measurable values, wherein each analyte value range has a range width and extends between a lower range limit value and an upper range limit value, i.e., comprising all values of the measurement range between those two range limit values and, optionally one or both of the range limit values. The overall measurement range is portioned by a series of consecutive analyte value range. In particular, the series of analyte value ranges may cover the overall measurement range in such a way that adj acent analyte value ranges are separated by a range limit value and that all values of the overall measurement range belong to exactly one of the analyte value ranges. A corresponding analyte value range is a range comprising the respective numerical analyte result value or in other words, the numerical analyte result value falls within the corresponding analyte value range.

Steps (a) and (b) are executed in the listed order, which does not exclude that further steps are performed in between the steps. Furthermore, in some embodiments the method steps (a) and (b) are repeated regularly, e.g., on demand by a user, wherein other steps may be performed before, in between or after each iteration of steps (a) and (b) or before, in between or after some of the iterations. For example, the steps are repeated at different points in time to assess the analyte concentration of the subject at said different points in time. Thus, at different points in time a sample of the bodily fluid of the subject is taken/produced and applied to a reagent test region, an image is produced, and a numerical analyte result value is determined. It shall be understood that depending on the analyte, the test reagent and the color formation reaction there are limits to and/or conditions regarding the time in between taking a sample of the bodily fluid and/or applying the sample to the reagent test region and/or taking the image.

The analyte value ranges may be preset by the system, e.g. an application code running on the mobile device comprising the method, or by an HCP, or by a user, such as the subject. Right from the start or with time advancing the preset or current analyte value ranges may not be suitable for the subject. For example, depending on age, a level of fitness, other conditions or the like, a normal and/or target blood glucose concentration for the subject may be close to one of the preset range limit values, which would it make preferable for the subject to shift said range limit value.

"Adapting" a range limit value may refer to changing or shifting one or more current range limit values or, alternatively, to keeping or confirming one or more current range limit values or, alternatively, to setting one or more new range limit values, wherein the "adapting" depends on an analysis of a plurality of previously determined numerical analyte result values of the subject. It is understood that the previously determined numerical analyte result values may each be determined for a different sample of the bodily fluid of the subject and each sample being taken at different points in time. Furthermore, it is understood that the range widths of the two analyte value ranges separated by the adapted range limit value change correspondingly.

The adaption takes previous measurement results of the subject into account, e.g. by performing data analytics, and therewith enables an individualization of the analyte value ranges. The analyte value ranges may be adapted to better suit the subject's typical analyte concentrations, e.g. by shifting a range limit value if there are conflicts regarding said limit. A conflict regarding a range limit value may, for example, be defined as a clustering of concentration measurements close or too close to said range limit value. For example, the analysis may determine a distance of each of the considered numerical analyte result values to said range limit value and count the number of distances that are smaller than a preset threshold. For example, the preset threshold may take into account an error distribution of the measured numerical analyte result values. In particular, the preset threshold may take into account a probability to wrongly categorize an actual analyte concentration in a wrong, adjacent analyte value range due to the measurement error of the determined numerical analyte value range. A conflict could be defined as a number of determined distances exceeding another preset threshold.

The adaption may additionally be based on user's input. The user's input may comprise specific range limit values and/or analyte value ranges and/or specific information about the subject, such as age, previous conditions or the like. The user's input may be used as bases for determining one or more new shifted range limit values and/or as a boundary condition for the adaption step.

The analysis may be based on all analyte value ranges previously determined for the subject or on a certain selection of those analyte value ranges. The analysis may be based on a fixed, non-changing algorithm and/or on a learning algorithm.

For example, an adaption may be favorable if analyte concentrations of the subject, for example analyte concentrations correlated to certain events like fasting, after a meal or during or after physical exercise, typically cluster close to one of the range limit values. Due to measurement errors this may yield to an attribution of the neighboring analyte value range to some or many of the determined numerical analyte result values and thus to communicating constantly switching analyte value ranges. With a shift of said range limit value, a more consistent communication of measurement results is ensured.

In one embodiment, step (c) is triggered off automatically by an amount of iterations of step (a) within a preset time period exceeding a trigger threshold and/or by an amount of numerical analyte result values determined within a preset time period and falling into a preset boarder range around one of the range limit values exceeds a trigger threshold.

"Boarder range" refers to a portion of the overall measurement range that is positioned around a range limit value, thus a range boarder. A range limit value falls within the boarder range if it is smaller than an upper range limit of the boarder range and bigger than a lower range limit of the boarder range. The boarder range may be positioned symmetrically or asymmetrically around the range limit value. The symmetry may be chosen due to the criticality of the specific range limit value, e.g. regarding the subject's health, and of identifying a state classified in either of the adjoining analyte value ranges. The boarder range may be previously set and/or adjustable and may be different regarding width and symmetry for some or all of the range limit values. The time period may as well be preset by a provider or user or person associated with the user, such as an HCP, in a set-up step, and may be fixed afterwards or may be adjustable by the subject, an HCP and/or any suitable person or facility.

Counting numerical values falling within a boarder range may be an indicator of conflict and may therefore represent a suitable trigger for a survey of at least the affected range limit value and, where appropriate, an adaption of range limit values.

A higher number of range limit values provides a broader data base for the adaption step (c). For example, a counter may be incremented with every execution of step (a) or with every determined numerical analyte result value that falls within any boarder range. Alternatively, there is a distinct counter regarding every boarder range. The counter or counters may be reset when an adaption step is performed.

In one embodiment, step (c) is triggered off automatically based on analyzing previously determined numerical analyte result values.

Thus, the previously determined numerical analyte result values are constantly monitored and if an analysis of those values shows any triggering events, e.g. conflicts regarding one or more of the range limit values, step (c) is triggered. The analysis may be the same as the analysis done in step (c). In particular, the analysis done before triggering may be used as bases for the adaption step itself, thus for the shifting of a range limit value.

In one embodiment, step (c) is triggered off manually via an input device of the mobile device or via a trigger message received by the mobile device via an input port of the mobile device.

A manual triggering of the adaption step may, for example, be requested by the subject or another user of the method, e.g. a parent, or by an HCP.

In one embodiment, step (c) includes analyzing the plurality of numerical analyte result values with regard to a distance to one or some or all of the range limit values.

If too many numerical analyte result values are too close to one of the range limit values, this might indicate a conflict and be a trigger for shifting the affected range limit value(s). Therefore, the distance is an appropriate measure for a range limit adaption. The distance may be determined by calculating a distance of each of the considered numerical analyte result values or by calculating a distance of a mean or a median or another representative value determined for the considered numerical analyte result values.

In one embodiment, the adaption of the range limit value is based on numerical analyte result values of the subject determined in a preset time period and/or on numerical analyte result values of the subject falling into a preset boarder range around a range limit value.

Limiting the numerical analyte result values used as bases for the adaption to a preset time period may help to exclude data that is too old to be significant and/or to limit the analysis on more relevant measurements and/or to accelerate the adaption step. A limitation to numerical analyte result values falling into one or more preset boarder ranges each around respective range limit values may help to limit the analysis to the most relevant and/or to the most critical numerical analyte result values regarding a decision about the plausibility and/or suitability of the range limit values for the subject.

In one embodiment, the adaption of the range limit value is based on one or more of a group of a mean, a median, a standard deviation, a coefficient of variation and a skewness determined for all or a selection of the previously determined numerical analyte result values of the subject.

In this embodiment, to adapt a range limit value based on previously determined numerical analyte result values, a representative quantity is formed from all or a selection of the previously determined numerical analyte result values. The representative quantity may be representative of the values and/or of their quality. For example, the representative quantity may be a mean or a standard deviation or any of the other listed quantities or any other quantity likewise representative of the considered numerical analyte result values. For the calculation, all result values already determined are taken into account, or only those result values determined in a preset time period, or related to predefined events, such as before or after a meal or after a physical exercise or any other event impacting the concentration of the analyte, or those determined in a preset value range, for example close to a critical value and/or close to one of the range limit values, or all those numerical analyte result values not yet taken into account by an earlier adaption step or any other suitable selection.

In one embodiment, the adaption of the range limit value is based on meeting at least one preset boundary condition.

To set certain limits to the variation or shifting of range limit values, for example by way of preset boundary conditions, may help to enhance the security of the method and/or to prevent malfunctioning of the method. The boundary conditions may be motivated by properties of the analyte, by the function of the analyte for the subject and/or by properties of determination process, i.e., the measuring process.

In one embodiment, at least one boundary condition is chosen from the group of not reducing a range width below a width threshold value, not reducing an upper range limit value of a lowest analyte value range below a lower limit threshold, and not increasing an upper range limit value of a lowest analyte value range above an upper limit threshold,

If certain concentration values of the analyte are particularly critical, e.g. regarding the subjects health, it may be reasonable to set upper or lower limits to a shifting of a range limit value close to or identical with the critical concentration value.

For example, if the analyte is blood glucose, it may be reasonable to ensure that a lowest analyte value range has an upper range limit value of at least 70 mg/dl and/or of 80 mg/dl or 90 mg/dl at most. In another embodiment with the analyte being glucose, reducing a range width may be limited to a minimum range width of 50 mg/dl or of 40 mg/dl.

Another additional or alternative boundary condition may be to always shift a range limit value in favor of "good" category ranges, thus shifting the range limit value in such a way that the width of the adjacent analyte value ranges that is categorized more "in range"/"better" is extended and the width of the adjacent analyte value range that is categorized more "out of range"/"worse" is reduced.

In one embodiment, the adaption of the range limit value includes receiving one or multiple range limit values and/or one or multiple analyte value ranges and/or other subject specific information from which range limit values are derivable.

Additionally to the previously determined numerical analyte result values of the subject, further information may be considered when adapting range limit values. The information may be received via wired or wireless communication or via an input device comprised by the mobile device. For example, the adaption may additionally be based on other subject specific information such as age, gender, other health conditions or any other relevant information about the subject. Deriving range limit values may comprise choosing from multiple preset series of analyte value ranges or looking up preset adaption rules.

In one embodiment, the adaption step includes asking a user to confirm or to refuse the set range limit values.

The user may be the subject, i.e., patient, or an HCT or any other suitable person, e.g. a parent.

In one embodiment, the corresponding analyte value range and/or a message associated with the corresponding analyte value range is displayed on a display device of the mobile device;

An associated message or, in other words, a corresponding message may be any message suitable to inform the user of the analyte value range determined regarding the subject's current analyte concentration.

In another aspect, a computer program is disclosed including computer-executable instructions for performing the method described above.

In a further aspect, a non-transitory computer-readable storage medium is disclosed, the computer-readable storage medium including instructions that when executed by a mobile device, cause the mobile device to perform the method as described above.

In another aspect, a mobile device is disclosed including a processing device, a display device, a camera, a display device, and a memory storing instructions that, when executed by the processing device, configure the mobile device to perform the method as described above.

In a further aspect, a kit for determining a concentration of an analyte in a bodily fluid, the kit includes at least one mobile device described above and at least one optical test strip having at least one test field.

Other technical features of the different aspects of invention may be readily apparent to one skilled in the art from the following figures, descriptions, and claims.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates the subject method in accordance with a first embodiment.
FIG. 2 shows a table of analyte value ranges in accordance with one embodiment.

### DETAILED DESCRIPTION

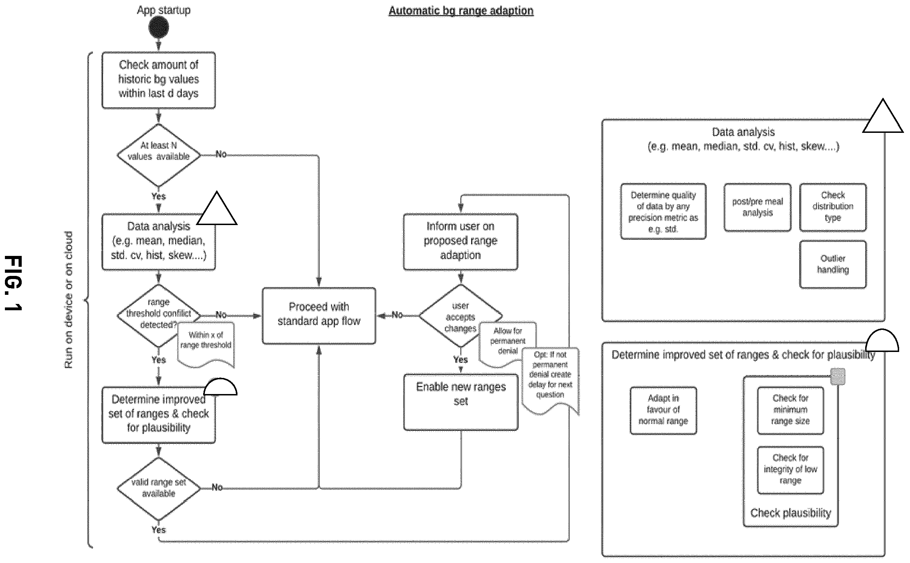

In FIG. 1 a first embodiment of a method of individualizing analyte value ranges used to categorize a concentration of an analyte in a sample of a bodily fluid of a subject applied to a reagent test region and determined using a mobile device is illustrated. The method, for example as an application on a mobile device such as a smartphone, is started, for example by the user or by any other trigger, such as a trigger message, a preset time schedule or the like. The app startup is indicated by a dot in the figure.

In the illustrated embodiment as a first step each time the application is started an amount of numerical analyte result values determined within a preset number of previous days, thus within preset time period, is checked. If the number of numerical analyte result values in the defined time period does not reach or exceed a preset threshold number N, the method proceeds with method steps (a) and (b) which is indicated as "standard app flow" in the figure. Steps (a) and (b) comprise the determining of a numerical analyte result value from an image of the color formation of the reagent test region after applying the subject's bodily fluid sample and the attributing of the numerical analyte result value to a corresponding analyte value range, wherein the analyte value range is one of a series of consecutive analyte value ranges portioning an overall measurement range. Each of the analyte value ranges comprises a range width extending between a lower range limit value and an upper range limit value, wherein adjacent analyte value ranges are separated by a mutual range limit value and the mutual range limit value is comprised by one of the adjacent analyte value ranges while the other adjacent range limit value comprises all values smaller or bigger than the range limit value, whichever is applicable.

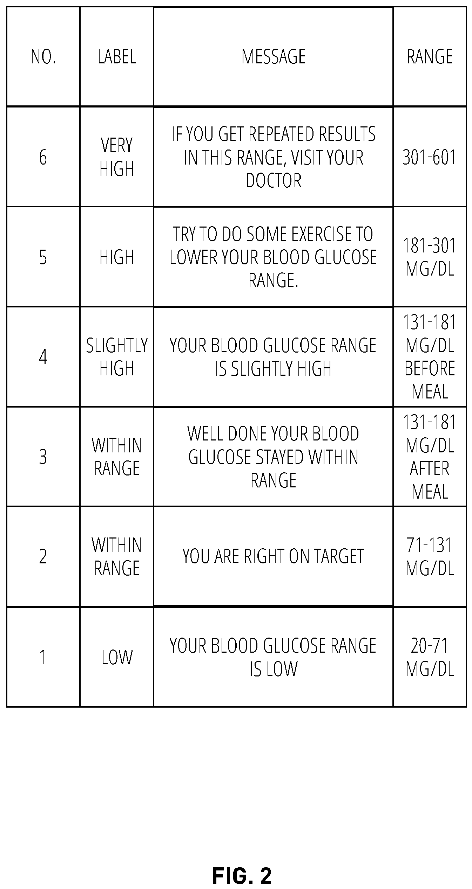

An example of a series of analyte value ranges for blood glucose is given in FIG. 2.

If the amount of numerical analyte result values determined within the preset time period reaches or exceeds the threshold number N, the method proceeds with the adaption step.

In the illustrated embodiment the adaption step starts with an analysis of the numerical analyte result values determined within the preset time period, for example (indicated by triangle) comprising the calculation of a mean and/or a median and/or a standard deviation and/or a coefficient of variation and/or a skewness for all or a selection of said numerical analyte result values.

The analysis may also comprise handling of outliers. The selection may be made with regard to a distance of the numerical analyte result value to one of the range limit values and/or to a numerical analyte result value being linked to a specific event, such as pre-meal or post-meal, and/or a time of determination of a numerical analyte result value.

In the illustrated embodiment the numerical analyte result values are analyzed with regard to a conflict with one or with any of the range limits, wherein a conflict may be identified by too many of the considered numerical analyte result values being too close to one of the range limit values. It shall be understood that being too close may be defined as a corresponding distance between a numerical analyte result value and a range limit value being smaller than a preset threshold. Correspondingly, "too many of the considered numerical analyte result values" may be defined as their number exceeding a corresponding preset threshold.

If no conflict is detected, the method proceeds with the standard app flow, i.e. with steps (a) and (b).

If a conflict is detected, the adaption step continues with determining an improved set of ranges by shifting at least one range limit value that is affected by the conflict. For example, a lower range limit value of a first analyte value range is decreased to extend the width of the first analyte value range to also cover lower analyte concentration values if a significant number of previously defined numerical analyte result values are accumulating too close to said lower range limit value. A width of an adjacent lower analyte value range is correspondingly reduced.

In the adaption step for any conflict detected, the affected range limit value is adapted yielding a new series of analyte value ranges defined by shifted and un-shifted range limit values, respectively, so that the analyte value ranges of the new series still fully cover and portion the overall measurement range.

In the illustrated embodiment the adaption step further comprises checking for plausibility. Such a plausibility check (semi circle) may comprise checking on boarder conditions, e.g. ensuring that all range widths still have a preset minimum width and/or that an upper range limit value of a certain analyte value range, e.g. a lowest analyte value range, is not lower than preset threshold and/or that a range width of a favorable analyte value range such as a range covering those analyte concentration values classified as normal is preferably enlarged and not decreased.

In the illustrated embodiment if the adapted range limit values and/or analyte value ranges do not pass the plausibility check those values/ranges are discarded and the method proceeds with the standard app flow, thus with steps (a) and (b) using the old analyte value ranges with the old range limit values that had been used before the adaption step.

If the analyte value ranges defined by the adapted range limit values and/or the adapted range limit values do pass the plausibility check the subject or any other user is informed about the new adapted analyte value ranges and asked to agree to them. If the user agrees, the method proceeds with the standard app flow, i.e. with steps (a) and (b) using the new adapted series of analyte value ranges.

If the user does not agree with the new series of analyte value ranges, the method proceeds with the standard app flow, thus with steps (a) and (b) using the old series of analyte value ranges and discards the new series of analyte value ranges with the adapted range limit values.

Optionally the adapted range limit values and/or series of analyte value ranges comprising certain adapted range limit values may be permanently denied by the user when asked to agree to the adaption. Thus, said range limit value or values permanently denied are saved and added as another boundary condition to the plausibility check step.

In another embodiment, those range limit values the user does not agree with are saved and added as another boundary condition to the plausibility check step for a certain preset amount of time.

In FIG. 2 a table is shown listing five different analyte value ranges portioning an overall measurement range ranging from 20 mg/dl to 601 mg/dl according to an embodiment with the analyte being blood glucose. To each numerical analyte result value determined in step (a) the corresponding analyte value range from the table is attributed in step (b). The range that corresponds is the range that contains the numerical analyte result value.

The numerical analyte result values covered by each analyte value range are given in the last column, wherein each mutual range limit value separating adjacent analyte value ranges is contained by one of the adjacent ranges while the other adjacent range comprises all smaller or larger values, respectively. For example, all analyte value ranges comprise the respective lower range limit value given in the fourth column and all values from said lower range limit value and smaller than the given upper range limit value. Thus, the first analyte value range is defined as ≥ 20 mg/dl to < 71 mg/dl.

To communicate the measurement result to the user, thus the subject or any other person, the measurement range is displayed on a display device, such as a display device of the mobile device. The measurement range may be communicated by displaying the corresponding range limit values given in the fourth column or by displaying the name, e.g. as given in the second column. Alternatively, a corresponding message may be displayed, for example the messages given in the third column.

## Claims

1. A method of individualizing analyte value ranges used to categorize a concentration of an analyte in a sample of a bodily fluid of a subject applied to a reagent test region and determined using a mobile device having a processing device, the method comprising the steps of:
(a) determining by the processing device from an image of a color formation of the reagent test region a numerical analyte result value within an overall measurement range;
(b) attributing the numerical analyte result value to a corresponding analyte value range of a series of consecutive analyte value ranges portioning the overall measurement range, each analyte value range with a range width extending between range limit values, each range limit value separating adjacent analyte value ranges;
(c) adapting by the processing device or any other processing device one or more range limit values based on a plurality of numerical analyte result values previously determined for different samples of the subject's bodily fluid taken at different points in time.

2. The method of claim 1, wherein step (c) is triggered off automatically by an amount of iterations of step (a) within a preset time period exceeding a trigger threshold and/or by an amount of numerical analyte result values, determined within a preset time period and falling into a preset boarder range around one of the range limit values, exceeds a trigger threshold.

3. The method of claim 1, wherein step (c) is triggered off automatically based on analyzing previously determined numerical analyte result values.

4. The method of claim 1, wherein step (c) is triggered off manually via an input device of the mobile device or via a trigger message received by the mobile device via an input port of the mobile device.

5. The method of any one of claims 1 to 4, wherein step (c) comprises analyzing the plurality of numerical analyte result values with regard to a distance to one or some or all of the range limit values.

6. The method of any one of claims 1 to 5, wherein the adaption of the range limit value is based on numerical analyte result values of the subject determined in a preset time period and/or on numerical analyte result values of the subject falling into a preset boarder range around a range limit value.

7. The method of any one of claims 1 to 6, wherein the adaption of the range limit value is based on one or more of a group of a mean, a median, a standard deviation, a coefficient of variation and a skewness determined for all or a selection of the previously determined numerical analyte result values of the subject.

8. The method of any one of claims 1 to 7, wherein the adaption of the range limit value is based on meeting at least one preset boundary condition.

9. The method of claim 8, wherein at least one boundary condition is chosen from the group of
- not reducing a range width below a width threshold value,
- not reducing an upper range limit value of a lowest analyte value range below a lower limit threshold;
- not increasing an upper range limit value of a lowest analyte value range above an upper limit threshold;

10. The method of any one of claims 1 to 9, wherein the adaption of the range limit value comprises receiving one or multiple range limit values and/or one or multiple analyte value ranges and/or other subject specific information from which range limit values are derivable.

11. The method of any one of claims 1 to 10, wherein the adaption step comprises asking a user to confirm or to refuse the set range limit values.

12. The method of any one of claims 1 to 11, wherein the corresponding analyte value range and/or a message associated with the corresponding analyte value range is displayed on a display device of the mobile device;

13. A computer program including computer-executable instructions for performing the method according to any one of claims 1 to 11.

14. A non-transitory computer-readable storage medium, the computer-readable storage medium including instructions that when executed by a mobile device having a camera, a processing device, and a display device cause the mobile device to perform the method according to any one of claims 1 to 11.

15. A mobile device comprising:
a processing device;
a camera;
a display device, and
a memory storing instructions that, when executed by the processor, configure the mobile device to to perform the method according to any one of claims 1 to 11.

16. A kit for determining a concentration of an analyte in a bodily fluid, the kit comprising at least one mobile device according to claim 15 and at least one optical test strip having at least one test field.
